# EUROPÄISCHE PATENTSCHRIFT

(11) **EP 0 789 528 B1**
(45) Veröffentlichungstag und Bekanntmachung des Hinweises auf die Patenterteilung: **21.11.2001**
(21) Anmeldenummer: 96928471.0
(22) Anmeldetag: 16.08.1996
(51) Int. Cl.: A61B 1/267, A61B 1/32, A61B 1/24

(54) **STÜTZENDOSKOP, INSBESONDERE FÜR UNTERSUCHUNGEN IM BEREICH DES EPI-, ORO- UND/ODER HYPOPHARYNX UND/ODER LARYNX**
SUPPORTING ENDOSCOPE ESPECIALLY FOR EXAMINATIONS OF REGIONS OF EPIPHARYNX, OROPHARYNX AND/OR HYPOPHARYNX AND/OR LARYNX
ENDOSCOPE DE SOUTIEN, NOTAMMENT POUR EXAMENS DE LA REGION DE L'EPIPHARYNX, DE L'OROPHARYNX ET/OU DE L'HYPOPHARYNX ET/OU DU LARYNX

(30) Priorität: 30.08.1995 DE 19532098
(43) Veröffentlichungstag der Anmeldung: 20.08.1997
(73) Patentinhaber: Explorent Chirurgische Instrumente Gmbh, 78532 Tuttlingen (DE)
(72) Erfinder: KASTENBAUER, Ernst, D-82335 Berg (DE); FEYH, Jens, D-81377 München (DE)
(74) Vertreter: Götz, Georg, Dipl.-Ing.
(86) Internationale Anmeldenummer: EP9603613
(87) Internationale Veröffentlichungsnummer: WO9707732

(56) Entgegenhaltungen:
- GB-A- 2 189 148
- US-A- 2 969 059
- US-A- 3 320 948
- US-A- 4 151 837
- US-A- 4 300 541
- US-A- 5 063 908

## Beschreibung

Die Erfindung richtet sich auf eine Vorrichtung für endoskopische Untersuchungen und/oder Operationen, insbesondere im Bereich des Epi-, Oro-, Meso- und/oder Hypopharynx und/oder des Larynx.

In dem oben erwähnten Bereich des Pharynx und des Larynx kann eine Vielzahl von krankhaften Veränderungen auftreten, welche nicht selten eine operative Therapie erfordern. So treten hier neben gutartigen Tumoren auch Karzinome des Pharynx, bspw. das pharyngeale Lymphoepitheliom, und des Larynx, bspw. in Form des Kehlkopfkarzinoms, auf. Je nach dem Ort dieser Tumore wird dabei eine Pharyngektomie oder eine Laryngektomie durchgeführt. Zur Diagnose von Tumoren im Bereich des Pharynx wird ein sog. Laryngoskop verwendet, d.i. ein Endoskop mit einem Spatel zum Anpressen der Zunge an den Mundboden sowie gegebenenfalls einem Kehlkopfspatel. Durch ein derartiges Laryngoskop kann auch mittels Laserchirurgie operiert werden, jedoch gestaltet sich sowohl die Diagnose wie auch die Therapie in manchen Fällen schwierig, bspw. dann, wenn sich ein Tumor in den Valleculae befindet; dies resultiert aus dem Röhrendurchmesser eines derartigen Laryngoskops. Im Bereich des Übergangs vom Oro- bzw. Mesopharynx auf den Hypopharynx sind mit einer erhöhten Inzidenz maligne Tumore anzutreffen, deren laserchirurgische Entfernung infolge des intraoperativ intermittierenden Einsatzes von Instrumenten zur Darstellung der entsprechenden anatomischen Region bislang deutlich erschwert ist. Darüber hinaus gibt es eine Reihe weiterer Probleme insbesondere im Bereich des Oro- und Mesopharynx, welche einen operativen Eingriff notwendig machen, bspw. eine Tonsillektomie. In all diesen Fällen kann eine Diagnose und/oder ein operativer Eingriff zwar prinzipiell durch den Pharynx hindurch vorgenommen werden; wegen dessen gewundenen Aufbaus und der begrenzten Möglichkeit eines Patienten, seinen Mund weit zu öffnen, ist es jedoch äußerst schwierig, entsprechende Untersuchungs- und/oder Operationsistrumente bis zu der betreffenden Stelle einzuführen.

Ähnliche Schwierigkeiten treten auch im Rahmen endoskopisch-diagnostischer Maßnahmen und/oder operativer Therapien im Bereich anderer Körperorgane auf, bspw. bei der Kardiochirurgie, Laparoskopie, Neurochirurgie oder Gynäkologie. Auch hier ist oftmals der Zugangsweg sehr beengt, sodaß Untersuchungen und/oder Operationen stark behindert werden.

Eine Vorrichtung gemäß dem Stand der Technik ist durch US-A-2 969 059 bekannt. Der Oberbegriff des Anspruchs 1 entspricht dieser Vorreröffentlichung.

Aus diesem Nachteil des bekannten Stands der Technik resultiert das die Erfindung initiierende Problem, eine Vorrichtung zu schaffen, mit der endoskopische Untersuchungen und/oder Operationen, insbesondere im Bereich des Pharynx und des Larynx, vorgenommen werden können, ohne daß hierbei ein weitgehend starres Instrument mit einem großen Rohrquerschnitt, wie dies bei herkömmlichen Endoskopen der Fall ist, bis zu der betreffenden Stelle vorgeschoben werden muß, und ohne daß eine Operation gegebenenfalls ohne direkte Sicht durchgeführt werden muß. Ferner besteht ein Bedürfnis nach einer Vergrößerung des Zugangswegs und nach einer dynamischen Einstellung aller anatomischen Bezirke der betreffenden Region, damit die Operation präziser und dadurch sicherer wird.

Die Lösung dieses Problems gelingt bei einer gattungsgemäßen Vorrichtung durch eine erfindungsgemäße Konstruktion, wie sie im kennzeichnenden Teil des Hauptanspruchs beschrieben ist. Das Kernstück der erfindungsgemäßen Vorrichtung bildet ein steifes Gerüst, welches bspw. mit einer Bruststütze fixiert werden kann und zur Befestigung einer Vielzahl von Instrumenten dient, insbesondere Sperr- und Spreizinstrumenten zur Offenhaltung des Mundes wie Zungen- und Mundwinkelspatel sowie einer Gaumenplatte, für Hilfsinstrumente wie Leucht- und Absaugeinrichtungen, für Untersuchungsinstrumente wie Kehlkopfspiegel sowie für Operationsinstrumente wie Faßzangen und Skalpelle für die Tonsillektomie oder für laserchirugische Instrumente. Durch die erfindungsgemäße Gaumenplatte kann der Mund eines Patienten weit auseinander gedrückt werden, ein Zungenspatel drückt die Zunge weitgehend aus dem Sichtbereich und kann gegebenenfalls mit einem Kehlkopfspatel kombiniert sein, der eine Anhebung der Epiglottis hervorruft und dadurch den Larynx öffnet. Mundwinkelspatel spreizen die Buccae lateral auseinander, um einen möglichst großen Zugang für Hilfsmittel und Instrumente zu schaffen. Als Hilfsmittel dienen insbesondere Leuchtmittel, Absaugeinrichtungen od. dgl., welche optimale Voraussetzungen für eine Diagnose oder Operation bilden. Schließlich können die Untersuchungs- und/oder Operationsinstrumente beweglich an dem erfindungsgemäßen Gerüst festgelegt werden, um einerseits deren Lage zu stabilisieren und andererseits exakte Bewegungen zu ermöglichen. Hierdurch kann die Präzision eines Eingriffs erhöht werden, so daß die Belastung eines Patienten herabgesetzt wird.

Der Halterung einer Vielzahl von Hilfsmitteln und/oder Instrumenten dienen ein oder mehrere langgestreckte, stabile Bügel, auf denen die betreffenden Mittel und/oder Instrumente anklemmbar oder verschiebbar sind. Erfindungsgemäß sind hierbei zwei zueinander etwa parallele Befestigungsbügel vorhanden, die einen Abstand von etwa 5 bis 10 cm aufweisen und während des Gebrauchs etwa parallel zu den Buccae eines Patienten verlaufen. Diese Bügel dienen u.a. zum Festklemmen je eines Mundwinkelspatels; um hierbei die bei einer Aufspreizbewegung auftretenden Kräfte formschlüssig aufnehmen zu können, sind die Befestigungsbügel mit einem polygonalen, insbesondere quadratischen Querschnitt versehen, auf denen die etwa hülsenförmigen Halterungsteile für einen Mundwinkelspatel formschlüssig aufgeschoben sind.

Diese beiden Befestigungsbügel sind durch einen etwa im Bereich des Mentums eines Patienten befindlichen Quersteg zu einem vorzugsweise U-förmigen, gegebenenfalls auch abgerundeten oder viereckigen Befestigungsgerüst und/oder-rahmen miteinander verbunden. Um dieses Gerüst an die Mund- sowie Mandibularbreite eines Patienten anpassen zu können, mögen die Befestigungsbügel gegenüber dem Quersteg um Drehachsen verstellbar ausgebildet sein, welche lotrecht zu der Grundfläche des U-förmigen Befestigungsgerüsts verlaufen. Diese Verstellmöglichkeit kann bspw. durch einen Schraub-Klemm-Mechanismus mit einer gleichzeitig als Drehgelenk fungierenden Klemmschraube realisiert sein.

Der Quersteg des U-förmigen Befestigungsgerüsts trägt außerdem ein Aufnahmemittel für einen Zungenspatel sowie ein Anschlußelement für eine Bruststütze. Dabei ist das Aufnahmemittel für den Zungenspatel derart angeordnet, daß die Längsachse des Zungenspatels etwa lotrecht zu der Grundebene der U-förmigen Befestigungsgerüsts verläuft, so daß die Befestigungsbügel nahe den Buccae eines Patienten verlaufen.

In der Praxis hat sich eine Anordnung bewährt, wobei das Aufnahmemittel für den Zungenspatel gegenüber dem Quersteg entlang einer zu den Befestigungsbügeln etwa parallelen Achse verschiebbar ist. Diese Anordnung eignet sich insbesondere für eine Weiterbildung der Erfindung, wobei auf den dem Quersteg gegenüberliegenden, freien Enden der Befestigungsbügel ein Querbügel mit einer Platte angeordnet ist, welche an dem Gaumen eines Patienten angelegt wird und dadurch die Position des U-förmigen Befestigungsgerüsts definiert. Wird nun das Aufnahmemittel für den Zungenspatel gegenüber dem Quersteg verschoben, so öffnet sich dadurch die Mandibula eines Patienten, wodurch eine funktionssichere Mundsperre bewerkstelligt ist. Um eine derartige Verschiebbarkeit zu ermöglichen, ist das Aufnahmemittel für den Zungenspatel an dem oberen Ende einer Säule angeordnet, welche durch ein Führungselement des Querstegs hindurchtritt und an ihrem unteren Ende gleichzeitig mit einem Anschlußelement für eine Bruststütze versehen sein kann. Mit Getriebemechanismen kann sowohl eine Verstellung als auch eine Arretierung der den Zungenspatel tragenden Säule herbeigeführt werden.

Eine Verstellmöglichkeit kann vorgesehen sein, um die effektive Länge des in den Pharynx ragenden Zungenspatels auf einen Patienten einstellen zu können. Hierzu kann der Zungenspatel mit seinem Griffteil in einem Führungsprofil aufgenommen sein, wobei mit einer Verstellschraube die Verschiebeposition des Spatels vorgegeben werden kann.

Die Erfindung verwendet eigens für dieses Befestigungsgerüst konstruierte Zungenspatel; diese zeichnen sich durch einen etwa löffelartigen Zungenteil und einen daran anschließenden, geradegestreckten Halterungsteil aus, der nicht oder nur geringfügig gegenüber dem Zungenteil geneigt ist, wobei das gesamte Zungenspatel aus einem steifen und festen Werkstoff, bspw. einer etwa 2,5 mm starken Edelstahlplatte gefertigt, insbesondere ausgestanzt und gebogen ist. Zur Längsverstellung ist der Halterungsteil mit gleichbleibendem Querschnitt ausgeführt und weist an seiner Unterseite eine durch quer verlaufende Einfräsungen gebildete Zahnreihe auf, in welche ein Verstell-Zahnrad des oben beschriebenen Aufnahmemittels eingreifen kann. Der Unterteil des löffelartigen Zungenteils kann mit einer reibungserhöhenden Profilierung versehen sein. Darüber hinaus sind unterschiedliche Formen des löffelartigen Zungenteils möglich: Bei einer Standardausführung ist dieses nahezu völlig eben und nur um wenige Grad gegenüber dem rückwärtigen Halterungsteil geneigt. Bei einer zweiten Ausführungsform kann das Zungenteil ähnlich dem Kehlkopfspatel eines konventionellen Laryngoskops bogenförmig zur Unterseite hin gekrümmt sein, so daß das freie Ende des Zungenteils mit dem Halterungsteil einen Winkel von etwa 15 bis 25° einschließt. Weiterhin ist es möglich, von der Löffelform abweichend an einer oder beiden Seiten des Zungenteils einen lateralen Ausschnitt vorzusehen, um Untersuchungen und/oder Operationen in den dadurch freigegebenen Bereichen zuzulassen. Außerdem ist es möglich, dem Zungenteil eine Wölbung in Querrichtung einzuprägen, derart, daß die lateralen Bereiche des Zungenteils nach oben zurückweichen, und Verletzungen der Lingua vermieden werden.

Die oben bereits angesprochene Gaumenplatte zur Abstützung des Palatums eines Patienten verfügt erfindungsgemäß über einen Bügel mit endseitig angeordneten Buchsen, die auf je ein freies Ende des U-förmigen Befestigungsgerüsts bis zu einem dort angeordneten Anschlagelement aufgeschoben werden können. Zur Aufnahme der Dentes incisivi und/oder des Alveolarkamms der Maxilla kann die Gaumenplatte in ihrer Längsrichtung konkav oder sattelförmig nach unten durchgewölbt sein.

Die von den Erfindern zum lateralen Aufspreizen der Buccae vorgesehenen Mundwinkelspatel haben eine langgestreckte, schmale Grundform. Zur Vermeidung von Verletzungen des Patienten ist die Vorderkante derartiger Mundwinkelspatel abgerundet und außerdem besteht ein derartiger Spatel aus einem begrenzt elastischen Werkstoff, bspw. einem weichmetallischen Blech mit einer Stärke von 1,5 bis 2 mm. Zur Einstellung des Spreizwinkel derartiger Mundwinkelspatel dient deren Befestigungselement, welches zu diesem Zweck mit einem Gelenk- und/oder Verstellmittel versehen ist. Derartige Mundwinkelspatel können auch zum Spreizen der lateralen Hypopharynxwand verwendet werden. Ein Mundwinkelspatel wird in diesem Bereich eingesetzt, indem er in seiner Führung weiter Richtung Hypopharynx vorgeschoben wird, und leistet dort insofern wertvolle Hilfe, als der Sinus piriformis, in welchem etwa 90 % aller Hypopharynxkarzinome beheimatet sind, während der Operation aufgehalten wird.

Erfindungsgemäß ist weiterhin ein Leuchtmittel vorgesehen, das aus einem Linsensystem mit einem rückwärtigen Schraubanschluß für ein Lichtfaserkabel gebildet sein kann und vermittels eines an dem Gehäuse dieses Elements angeordneten Kugelgelenks mit einer Klammer zum Festklemmen an einem der beiden Befestigungsbügel verbunden ist. Eine ähnliche Klammer kann anstelle des Leuchtmittels und/oder Kugelgelenks eine zweite Befestigungsklammer aufweisen, in der bspw. ein Absaugschlauch oder ein Instrument eingelegt und festgeklemmt werden kann.

Weitere Einzelheiten, Merkmale und Vorteile auf der Basis der Erfindung ergeben sich aus der folgenden Beschreibung eines bevorzugten Ausführungsbeispiels der Erfindung sowie anhand der Zeichnung. Diese zeigt in:
- Fig. 1: eine axonometrische Ansicht einer erfindungsgemäßen Vorrichtung, teilweise abgebrochen;
- Fig. 2: eine Seitenansicht auf die Figur 1 in Richtung des Pfeils II, teilweise im Schnitt;
- Fig. 3: die Unterseite des aus der Vorrichtung gemäß Figuren 1 und 2 gelösten Zungenspatel;
- Figuren 4a, 4b: weitere Zungenspatel mit abweichender Form des Zungenteils in einer der Figur 3 entsprechenden Darstellung;
- Fig. 5: einen gegenüber den Figuren 3 bis 4b geringfügig abgewandelten Zungenspatel in der Seitenansicht;
- Fig. 6: einen weiteren, erfindungsgemäßen Zungenspatel in der Draufsicht;
- Fig. 7: eine Seitenansicht des Zungenspatels aus Figur 6;
- Fig. 8: eine axonometrische Darstellung des linken Mundwinkelspatels aus Figur 1 in einem vergrößerten Maßstab, so daß das Aufnahme- und Spreizmittel deutlicher zu erkennen ist;
- Fig. 9: eine Draufsicht auf die Figur 8 in Richtung des Pfeils IX;
- Fig. 10: eine Seitenansicht auf das Aufnahmemittel der Figur 8 in Richtung des Pfeils X;
- Fig. 11: ein weiteres Aufnahmemittel zur Festlegung eines Instruments an der erfindungsgemäßen Vorrichtung in einer axonometrischen Darstellung;
- Fig. 12: eine Draufsicht auf die Figur 11 in Richtung des Pfeils XII, teilweise im Schnitt; sowie
- Fig. 13: eine Seitenansicht auf das Aufnahmemittel aus Figur 11 in Richtung des Pfeils XIII.

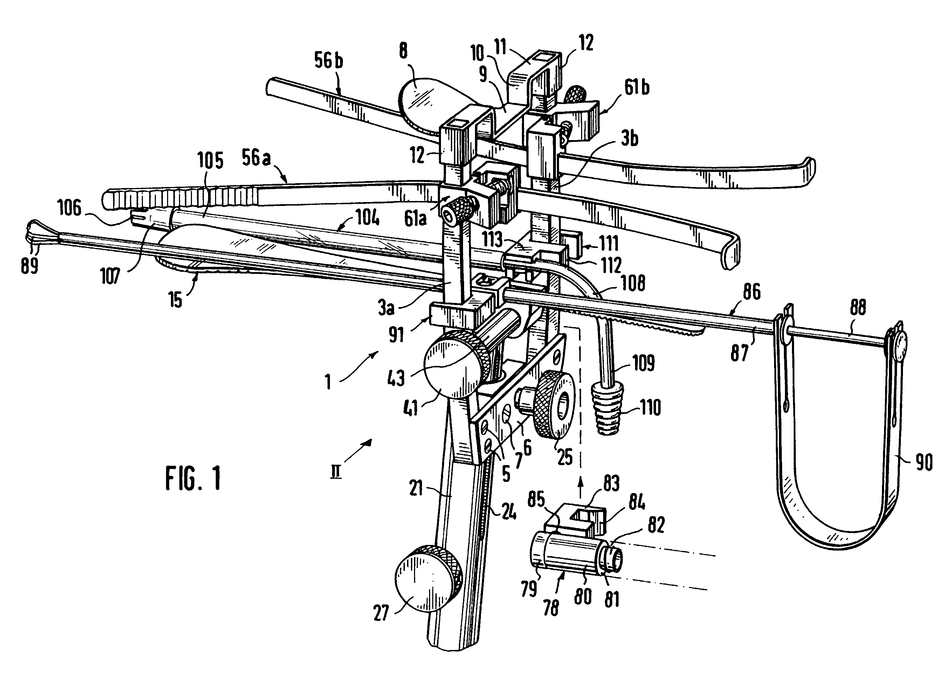

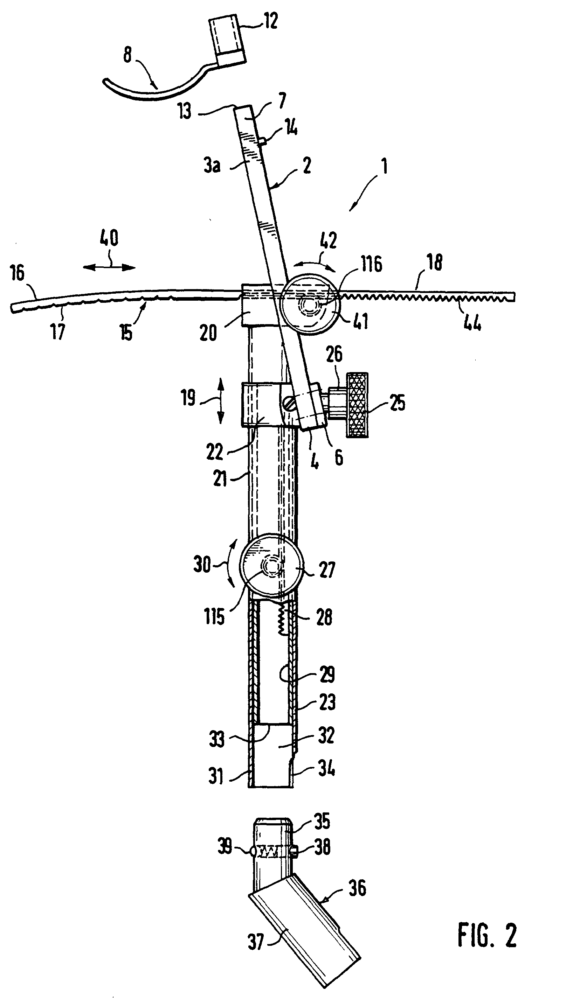

In Figur 1 ist ein erfindungsgemäßes Stütz-Epi-Oro-Hyphopharyngo-Laryngoskop 1 mit einer Vielzahl von angebauten Hilfsmitteln und Instrumenten wiedergegeben. Zur Erhöhung der Übersichtlichkeit soll daher zunächst auf Figur 2 Bezug genommen werden, bei der ein Großteil der Anbauteile weggelassen sind, so daß das eigentliche Gerüst 2 des erfindungsgemäßen Pharyngo-Laryngoskosps 1 deutlicher zu erkennen ist. Das Gerüst 2 umfaßt zwei Bügel 3a, 3b, welche einen gleichbleibenden, etwa quadratischen Querschnitt aufweisen. Diese beiden Bügel 3a, 3b sind leicht gekrümmt mit einem Krümmungsradius von etwa 25 bis 40 cm und an ihren unteren Enden 4 durch einen aufgeschraubten 5 Querbügel 6 zu einem ebenen etwa U-förmigen Gerüst 2 verbunden. Hierbei sind die Befestigungsschrauben 5 an dem Querbügel 6 derart angeordnet, daß die freien oberen Enden 7 der beiden Bügel 3a, 3b etwa parallel zueinander verlaufen, während die unteren Enden 4 aufgrund der bogenförmigen Gestalt zueinander konvergieren. Dieses in sich starre Gerüst 2 des erfindungsgemäßen Pharyngo-Laryngoskops 1 dient zur Fixierung einer Vielzahl von Sperr-, Spreiz- und Hilfsmitteln, Untersuchungs- und Operationsinstrumenten sowie einem Anschlußelement für eine Bruststütze.

Die in Figur 2 abgenommene, in Figur 1 dagegen montierte Gaumenplatte 8 hat eine etwa kreisförmige Grundfläche, welche in ihrer Längsrichtung etwa sattelförmig gewölbt ist, und dient zur Abstützung des rückwärtigen Bereichs des Palatum durum und/oder des Processus alveolaris maxillae bzw. der Dentes incisivi. Diese Gaumenplatte 8 ist zusammen mit einem sich an deren Vorderkante anschließenden Querbügel 9 aus einem etwa 2,5 mm starken Edelstahlblech ausgestanzt und damit stabil genug, um die zum Aufsperren der Maxilla notwendigen Kräfte verformungsfrei übertragen zu können. Der Querbügel 9 ist in seinem lateralen Bereich etwa in einem Abstand zur Medianebene, welcher der halben Breite der Gaumenplatte 8 entspricht, um ein Maß von etwa 1 cm nach oben abgekröpft 10, so daß in den distalen Bereichen 11 des Querbügels 9 unter dem Processus alveolaris maxillae sowie gegebenenfalls unter den Dentes incisivi hindurch die Cavitas oris propria optimal zugänglich ist. An den freien Enden der distalen Bereiche 11 ist sodann je ein hülsenförmiges Befestigungselement 12 angeordnet, insbesondere angeschweißt. Diese Befestigungshülsen 12 haben einen quadratischen Innenquerschnitt, der etwa dem Querschnitt der Befestigungsbügel 3a, 3b entspricht, und der durch den proximalen Teil und die beiden distalen Teile 11 des Querstegs 9 bestimmte Abstand zwischen den beiden Hülsen 12 entspricht dem Abstand zwischen den beiden oberen Enden 7 der beiden Querbügel 3a, 3b, so daß die Gaumenplatte 8 mit diesen Hülsen 12 auf das U-förmige Befestigungsgerüst aufgesteckt werden kann. Jeder der beiden Befestigungsbügel 3a, 3b weist in einem Abstand von etwa 1 cm unterhalb seiner oberen Stirnfläche 13 einen etwa horizontal auskragenden Fortsatz 14 auf, der als Anschlag für die Hülsen 12 dient und ein Herabrutschen der Gaumenplatte 8 entlang der Befestigungsbügel 3a, 3b verhindert. Die Längsachsen der beiden Hülsen 12 verlaufen etwa lotrecht zu der Grundfläche der Gaumenplatte 8.

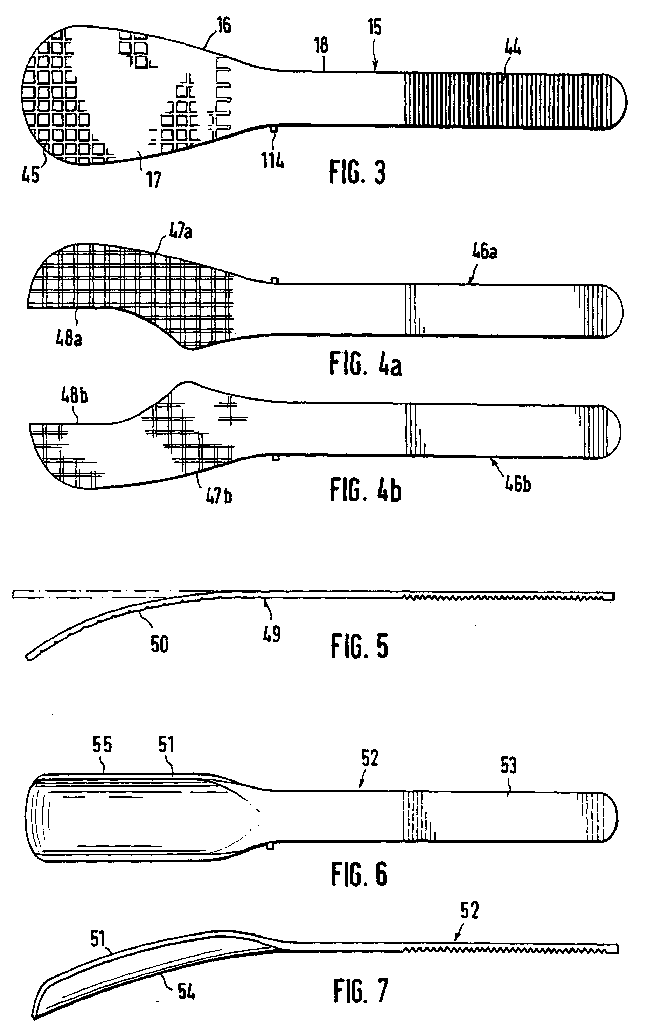

Die Gaumenplatte 8 findet als Sperrorgan ihr Komplement in einem Zungenspatel 15, womit die Lingua an den Mundboden gepreßt und gleichzeitig die Mandibula dadurch nach unten aufgesperrt wird. Wie in den Figuren 2 und 3 zu erkennen, hat der Zungenspatel 15 eine flache, nahezu ebene Form etwa mit dem Umriß eines Löffels. Der vordere oder Zungenteil 16 liegt mit seiner Unterseite 17 auf der Lingua des Patienten auf, während der rückwärtige, stielartige Bereich 18 an dem U-förmigen Befestigungsgerüst 2 gehaltert ist. Zum Ansetzen des erfindungsgemäßen Pharyngo-Laryngoskops 1 kann der Abstand zwischen der Gaumenplatte 8 und dem Zungenspatel 15 zunächst reduziert werden; danach wird der Abstand vergrößert, um die Cavitas oris aufzusperren. Diese Verschiebebewegung 19 des Zungenspatels 15 etwa lotrecht zu dessen Unterseite 17 wird dadurch erreicht, daß das Aufnahmemittel 20 für den Zungenspatel 15 auf einer Säule 21 angeordnet ist, welche in einer Hülse 22 längsverschiebbar ist. Diese Hülse 22 ist an dem Quersteg 6 derart festgeschraubt 7, daß ihre Längsachse und damit auch die Säule 21 in der medialen Ebene des U-förmigen Befestigungsgerüsts 2 sowie etwa parallel zu den beiden Befestigungsbügeln 3a, 3b verläuft. In Wirklichkeit ist die Grundebene des U-förmigen Befestigungsgerüsts 2 um einen Winkel von etwa 10° gegenüber der Längsachse der Säule 21 geneigt, um das Zurückweichen des Palatum durum während der Dorsalflexion des Schädels gegenüber der Lingua des Patienten durch eine etwa sekantenartige Orientierung des U-förmigen Gerüsts 2 zumindest teilweise zu kompensieren.

Zur Arretierung der Säule 21 in jeder beliebigen Verschiebestellung gegenüber der hülsenförmigen Halterung 22 ist an der dem Quersteg 6 zugewandten Seite 23 der Säule 21 ein Längsschlitz 24 vorgesehen, der von dem Schaft einer mit einem gerändelten Kopf 25 versehenen Schraube 26 durchgriffen wird, die außerdem in eine Bohrung des Querstegs 6 sowie des daran grenzenden Bereichs des hülsenförmigen Elements 22 eingesetzt ist. Diese Schraube ist in einem etwa zylindrischen Klemmelement eingeschraubt, welches sich innerhalb der Säule 21 befindet und im Falle der Arretierung von der Schraube 26 an den vorderen Bereich 23 der Säule 21 herangezogen wird, um durch Reibschluß eine Fixierung der Säule zu bewirken. Vor der Arretierung der Säule 21 in jeder beliebigen Zwischenposition ist eine definierte Verstellmöglichkeit 19 wichtig, um die Cavitas oris sachte und dennoch energisch aufsperren zu können. Hierzu dient eine zweite, gerändelte Schraube 27 vorgesehen, welche eine seitliche Bohrung der Säule 21 durchgreift. Diese Schraube ist jedoch an ihrem Schaft nicht mit einem Gewinde versehen, wie dies allgemein üblich ist, sondern mit einer ritzelartigen Zahnprofilierung 115, welche mit einer innerhalb der Säule 21 angeordneten Zahnstange 28 kämmt. Diese Zahnstange 28 ist an dem oben bereits erwähnten, zylindrischen Klemmteil, welches von der Schraube 26 zur Arretierung an die vordere 23 Innenseite 29 der Säule 21 angepreßt wird, unverrückbar verbunden, so daß durch Verschiebung der Zahnstange 28 infolge einer Drehung 30 der gerändelten Verstellschraube 27 bei gelöster Arretierschraube 26 auch das zylindrische Klemmteil und damit auch das Führungsteil 22 mitsamt dem Quersteg 6 gegenüber der Säule 21 verschoben 19 wird. Wird demnach die Rändelschraube 27 gemäß Figur 2 im Uhrzeigersinn gedreht 30, fährt 19 die Säule 21 gegenüber dem U-förmigen Gerüst 2 nach unten, was gleichbedeutend ist mit einer Anhebung der Gaumenplatte 8 gegenüber dem Zungenspatel 15 und einer Aufsperrung der Cavitas oris des Patienten.

Da sich hierbei der Abstand der Mandibula gegenüber dem Thorax kaum ändert, kann eine Bruststütze an dem unteren Ende 31 der Säule 21 angeschlossen werden. Dieses Säulenende 31 weist zu diesem Zweck eine buchsenartige Höhlung 32 mit einer inneren, rundumlaufenden Anschlagschulter 33 sowie eine achsparallele Einkerbung 34 auf. Diese Aufnahme 32 entspricht dem Standardzapfen handelsüblicher Bruststützen, wobei die Einkerbung 34 zum Einlegen einer in dem Zapfen der Bruststütze angeschraubten Klemmschraube dient. Zwar sind konventionelle Bruststützen meist mit einem verstellbaren Gelenk ausgerüstet, damit eine Anpassung an den jeweiligen Einsatzfall möglich ist. Die Verstellbarkeit eines derartigen Gelenks ist jedoch oftmals begrenzt, da derartige Stützen für den Anschluß an herkömmliche und damit anders konstruierte Laryngoskope optimiert sind. Um hier einen evtl. auftretenden Winkelunterschied zu kompensieren, sieht die Erfindung ein Kniestück 36 vor, welches einen Zapfen 35 zum Einstecken in die Buchse 32 einerseits sowie ein Buchsenelement 37 zur Verbindung mit der Bruststütze andererseits aufweist. Dabei schließen die beiden Teile 35, 37 des Kniestücks 36 einen Winkel von etwa 140° ein. Ein seitlicher Fortsatz 38 des Zapfens 35 greift in die Einkerbung 34 der Buchse 32 des erfindungsgemäßen Pharyngo-Laryngoskops 1 ein und vermeidet dadurch eine Verdrehung des Kniestücks 36. Dem Fortsatz 38 diametral gegenüberliegend ist eine abgefederte Kugel 39 zur Erzeugung eines Reibschlusses zwischen dem Zapfen 35 und der Buchse 32 angeordnet.

Vor dem Auseinandersperren der Cavitas oris ist es zunächst erforderlich, die Länge des Zungenspatels 15 auf den betreffenden Patienten einzustellen. Zu diesem Zweck ist der Zungenspatel 15 in der Aufnahme 20 entlang seiner Längsachse verschiebbar 40 aufgenommen. Hierzu ist das Aufnahmemittel 20 als den Spatelgriff 18 unter-, um- und zumindest teilweise übergreifende Schiene ausgebildet mit einem konstanten Profilquerschnitt, der etwa komplementär zu dem Querschnitt des Spatelgriffs 18 geformt ist. Zur Verstellung 40 wird eine Rändelschraube 41 betätigt 42, deren Drehachse 43 das Aufnahmeteil 20 etwa parallel zu dem Quersteg 6 durchsetzt. Der Schaft 43 hat überdies eine Länge, welche größer ist als der halbe Abstand zwischen den beiden Querbügeln 3a, 3b, so daß die Rändelschraube 41 außerhalb des Mundbereichs eines Patienten liegt und bequem betätigt werden kann. Der in dem Aufnahmemittel 20 befindliche Teil des Schraubmittels 41 ist ritzelförmig profiliert 116 und kämmt mit einer Zahnreihe 44, die an der Unterseite 17 des Spatelgriffs 18 eingefräst ist. Durch diesen Getriebemechanismus wird die Drehbewegung 42 der Rändelschraube 41 direkt in eine Längsverschiebung 40 des Zungenspatels 15 umgesetzt, der dadurch optimal an einen Patienten angepaßt werden kann. Eine Arretierung des Zungenspatels 15 in seiner Längsrichtung 40 wird nicht vorgenommen, damit sich beim Aufsperren 19 der Cavitas oris der Spatel 15 an die Anatomie des Patienten anpassen kann. Ein Anschlagfortsatz 114 vermeidet ein Verklemmen des verbreiterten Zungenteils 16 in dem Aufnahmemittel 20. Damit hierbei zwischen dem Zungenspatel 15 und der Lingua keine Versetzung auftritt, ist die Unterseite 17 des Zungenteils 16 des Spatels 15 mit einer raster- oder schachbrettartigen Profilierung 45 versehen.

In den Figuren 4 bis 7 sind weitere Ausführungsformen von Zungenspateln wiedergegeben. Hierbei entsprechen die Spatel 46a, 46b gemäß Figuren 4a, 4b weitgehend dem Zungenspatel 15 gem. Figur 3 mit der Ausnahme, daß der Zungenteil 47a, 47b an seiner rechten bzw. linken Seite mit einem Ausschnitt 48a, 48b versehen ist, um bestimmte Teile der Lingua zu Diagnose- und/oder Operationszwecken freizugeben.

In Figur 5 ist eine weitere Ausführungsform 49 eines Zungenspatels zu sehen, welche entweder die Grundform des Spatels 15 oder eines Spatels 46a, 46b aufweisen kann. Hier ist jedoch der Zungenteil 50 mit einer nach unten führenden Längswölbung versehen, damit der rückwärtige Bereich der Lingua gegen das Os hyoideum gedrückt wird, um einen optimalen Zugang zum Larynx zu schaffen.

Eine ähnliche Längskrümmung weist auch der Zungenteil 51 des in den Figuren 6 und 7 wiedergegebenen Zungenspatels 52 auf. Dieser ist sozusagen durch eine Kombination der beiden Zugenspatel 46a, 46b gebildet mit einem relativ schmalen Zungenteil 51, das kaum breiter ist als der Griff 53. Die Unterseite 54 des Zungenteils 51 ist darüber hinaus in Querrichtung konvex gewölbt, damit die Kanten 55 des Zungenteils 51 nicht in die Lingua einschneiden.

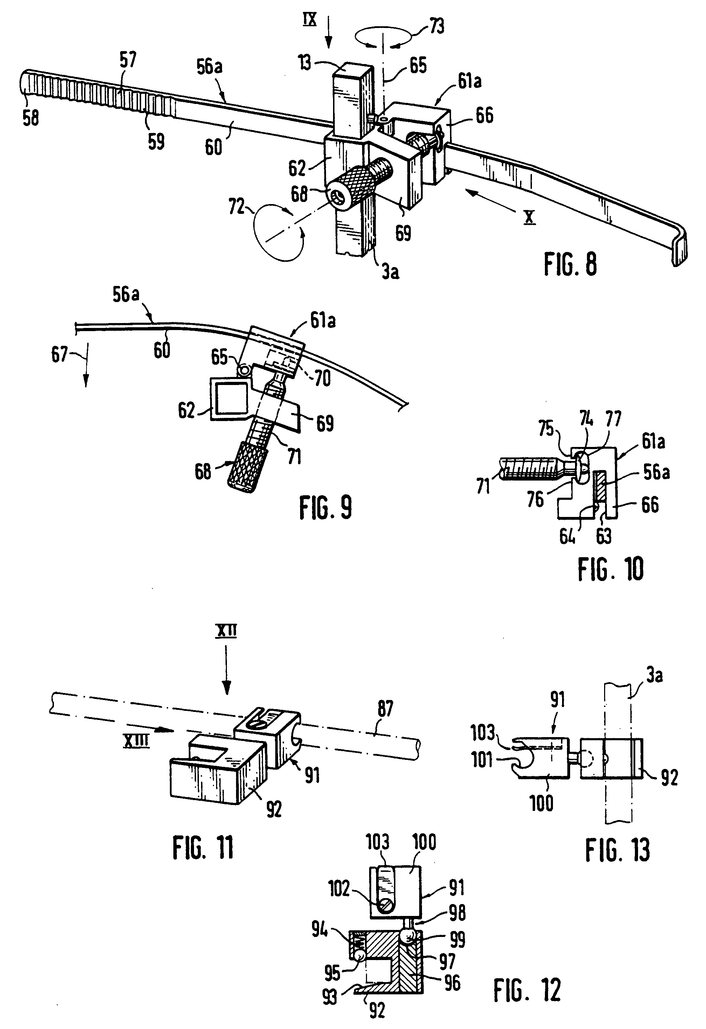

Ist solchermaßen vermittels der Gaumenplatte 8 und eines Zungenspatels 15 die Cavitas oris aufgesperrt, können die Buccae des Patienten seitlich aufgespreizt werden. Hierzu dienen zwei Mundwinkelspatel 56a, 56b, von denen der linke 56a in Figur 8 in vergrößertem Maßstab dargestellt ist. Man erkennt, daß jeder der beiden Mundwinkelspatel aus einem biegsamen Metallblech mit einer Stärke von etwa 2 mm besteht und eine Länge von etwa 20 bis 25 cm aufweist. Der vordere Bereich 57 ist mit einer abgerundeten Kante 58 sowie mit einer Profilierung 59 an der Außenseite 60 versehen.

Zum Fixieren und Aufspreizen dieser Mundwinkelspatel 56a, 56b dient je ein Aufnahmemittel 61a, 61b, welches mit einer Hülse 62 derselben Geometrie wie die Hülse 12 der Gaumenplatte 8 versehen ist. Diese Hülse 62 ist auf je einen der beiden Befestigungsbügel 3a, 3b aufgeschoben, und zwar unterhalb dessen Anschlagfortsatzes 14, so daß diese Aufnahmemittel 61a, 61b unverlierbar an dem U-förmigen Gerüst 2 gehalten sind.

Der Mundwinkelspatel 56a ist in einen Schlitz 63 des Aufnahmemittels 61a eingelegt, so daß die flache, profilierte 59 Außenseite 60 des Mundwinkelspatels 56a parallel zu der betreffenden Bucca des Patienten orientiert ist, und wird in dieser Position durch eine abgefederte Kugel 64 fixiert. Die in die Cavitas oris eingreifende Länge des Mundwinkelspatels 56a kann durch Verschieben innerhalb des Schlitzes 63 eingestellt werden. Zum Aufspreizen der beiden Mundwinkelspatel 56a, 56b dient eine zu der Längsachse des betreffenden Befestigungsbügels 3a, 3b parallele Drehachse 65, welche die Hülse 62 des Aufnahmemittels 61a gelenkig mit dem geschlitzten 63 Teil 66 des Aufnahmemittels 61a verbindet.

Das Gelenk 65 erlaubt das Aufspreizen 67 des vorderen Bereichs 57 des betreffenden Mundwinkelspatels 56a; zur exakten Einstellung des Spreizwinkels 67 dient eine weitere, gerändelte Schraube 68, welche in eine Gewindebohrung eines rückwärtigen, laschenartigen Fortsatzes 69 der Hülse 62 eingeschraubt ist. Die vordere Stirnseite 70 des Schraubenschafts 71 drückt gegen das geschlitzte Teil 66, so daß die Drehverstellung 72 der Rändelschraube 68 in eine Schwenkbewegung 73 des geschlitzten Aufnahmeteils 66 um die Drehachse 65 umgesetzt wird und schließlich zu einem Aufspreizen 67 des betreffenden Mundwinkelspatels 56a führt.

Um mit der Rändelschraube 68 auch ein Zusammenschwenken der beiden Mundwinkelspatel 56a, 56b bewirken zu können, ist im Bereich der vorderen Stirnseite 70 des Schraubenschafts 71 eine Kreisscheibe 74 angeformt, welche von zwei zueinander parallelen Hinterschneidungen 75, 76 des geschlitzten Teils 66 teilweise hintergriffen wird. Zwischen den beiden Hinterschneidungen 75, 76 verbleibt ein Schlitz zum Durchtritt des Schraubenschafts 71. Je nach Drehbewegung 72 der Rändelschraube 68 drückt daher entweder deren vordere Stirnfläche 70 gegen den Grund 77 des hinterschneidenden Bereichs (Aufspreizen 67), oder die Kreisscheibe 74 zieht über die Hinterschneidungen 75, 76 den geschlitzten Teil 66 gegen den laschenartigen Fortsatz 69, wobei der vordere Bereich 57 des Mundwinkelspatels 56a an die Medianebene des Patienten herangeschwenkt wird.

Nachdem solchermaßen die Cavitas oris aufgesperrt und die Buccae eines Patienten auseinandergespreizt sind, kann für eine Beleuchtung des Pharynx gesorgt werden. Hierzu verwendet der Arzt ein speziell zum Anbau an das erfindungsgemäße Befestigungsgerüst 2 konzipiertes Leuchtmittel 78, welches in Figur 1 wiedergegeben ist. Dieses besteht aus einer zylindrischen Fassung 79, in welcher ein Linsensystem eingepreßt ist. Auf einem rückwärtigen Gewindefortsatz der Fassung 79 ist ein Adapter 80 aufgeschraubt, der etwa die Form eines Zylindermantels aufweist und an seiner rückwärtigen Stirnseite 81 mit einem speziellen Anschlußgewinde 82 zum Aufschrauben eines Anschlußelements eines in Figur 1 nicht wiedergegebenen Lichtleitkabels versehen ist. An dem Fassungsteil 79 ist ein Befestigungsblock 83 angeordnet, der durch einen Schlitz 84 eine etwa U-förmige Gestalt erhält. Der Schlitz 84 hat etwa den Querschnitt eines Befestigungsbügels 3a, 3b, so daß das Leuchtmittel 78 an beiden Bügeln 3a, 3b wahlweise festgeklemmt werden kann. Zur Arretierung dient eine in Figur 1 nicht zu erkennende, abgefederte Kugel ähnlich der Kugel 39 des Knieteils 36 oder der Kugel 64 des Aufnahmemittels 61a. Weiterhin ist zwischen der Fassung 79 und dem Befestigungsblock 83 ein Kugelgelenk 85 vorgesehen, damit der Lichtkegel des Leuchtmittels 78 exakt justiert werden kann.

Nun kann eine Untersuchung oder Operation im Bereich des Pharynx oder des Larynx vorgenommen werden. Bei einer Operation wird oftmals eine Tumor-Faßzange 86 benötigt, wie sie ebenfalls in Figur 1 wiedergegeben ist. Diese besteht aus einem Hohlrohr 87, in welchem ein Stab 88 teleskopartig eingeschoben ist, der an seinem vorderen Ende unter Ausbildung mehrerer zangenartiger Greifelemente 89 in axialer Richtung unterteilt sowie radial aufgespreizt ist. Zwischen den rückwärtigen Enden des Teleskoprohrs 87 sowie der Teleskopstange 88 ist eine U-förmig gebogene Blattfeder 90 angeordnet, welche einerseits die Faßorgane 89 der Zange 86 selbsttätig schließt und andererseits als Griff dient.

Um mit einer derartigen Faßzange 86 exakt arbeiten zu können, wird diese ebenfalls in einem Punkt an dem Befestigungsgerüst 2 festgelegt. Hierzu dient ein weiteres Standardaufnahmemittel 91, welches in den Figuren 11 bis 13 wiedergegeben ist. Dieses besteht aus einem Befestigungsblock 92 zum Festlegen an einem der beiden Befestigungsbügel 3a, 3b, welcher identisch mit dem Befestigungsblock 83 des Leuchtmittels 78 ist. Man erkennt insbesondere in Figur 12 den Schlitz 93 zum Festklemmen an einem Befestigungsbügel 3a, 3b sowie die abgefederte 94 Kugel 95 zur reibschlüssigen Arretierung. Schließlich ist in dem Jochbereich 96 des querschnittlich U-förmigen Befestigungsblocks 92 die Pfanne 97 eines Kugelgelenks 98 eingeformt, deren Kugel 99 mit dem eigentlichen Aufnahmeblock 100 verbunden ist. Wie man aus Figur 13 entnehmen kann, ist der Aufnahmeblock 100 an seiner der Kugel 99 gegenüberliegenden Seite mit einem Schlitz 101 zum Einlegen des Teleskoprohrs 87 der Tumor-Faßzange 86 versehen. Eine an dem Aufnahmeblock 100 festgeschraubte 102 Blattfeder 103 federt radial in den Aufnahmeschlitz 101 hinein und legt dadurch das Teleskoprohr 87 in dem Schlitz 101 fest. Die Tumorfaßzange 86 ist dadurch in jeder Position selbstarretierend und kann dennoch in Längsrichtung innerhalb des Schlitzes 101 verschoben werden und außerdem um das Kugelgelenk 98 verschwenkt werden.

In ähnlicher Form wie die Tumor-Faßzange 86 kann in einem Standard-Aufnahmemittel 91 auch eine Vielzahl weiterer Untersuchungs- und/oder Operationsinstrumente eingesetzt werden, insbesondere auch ein Instrument zur Laserchirugie oder -koagulation. Bei derartigen operativen Lasereingriffen tritt naturgemäß eine starke Rauchentwicklung auf, welche die Sicht behindert und dadurch einen Großteil der Vorteile des erfindungsgemäßen Pharyngo-Laryngoskops 1 zunichte machen könnte. Deshalb sieht die Erfindung weiterhin eine Rauchabsaugeinrichtung 104 vor, wie sie in Figur 1 dargestellt ist. Diese umfaßt ein in die Cavitas oris ragendes Schlauchstück 105, auf dessen vorderes Ende eine sanft gekrümmte und mit Perforationen 106 versehene Hülse 107 zur Vermeidung von Verletzungen aufgeschoben ist. Der Schlauch 105 ist durch einen eingelegten und/oder eingegossenen Metalldraht versteift und mit seinem rückwärtigen Ende auf einen Rohrbogen 108 aufgesteckt, der an seinem rückwärtigen Ende 109 mit einem Schlauchanschlußstück 110 zum Aufschieben eines mit einer Absaugeinrichtung verbundenen Gummischlauchs versehen ist. Dieses Anschlußstück 110 verjüngt sich etwa kegelförmig zu seinem freien Ende, um das Aufschieben von Gummischläuchen unterschiedlichen Querschnitts zu ermöglichen. Weiterhin ist dieses Anschlußstück 110 an seiner Außenseite mit rundumlaufenden Wülsten versehen, um ein Herabrutschen eines aufgeschobenen Gummischlauchs zu vermeiden.

Zum Festklemmen einer derartigen Absaugvorrichtung 104 dient ein Aufnahmemittel 111, welches ähnlich dem Standard-Aufnahmemittel 91 einen Befestigungsblock 112 zum Anklemmen an einem der beiden Befestigungsbügel 3a, 3b aufweist, an dem jedoch ein geschlitzter Aufnahmeblock 113 starr, d.h., ohne Kugelgelenk, angeformt ist.

## Patentansprüche

1. Vorrichtung (1) für endoskopische Untersuchungen und Operationen im Bereich des Epi-, Oro-, Meso- oder Hypopharynx oder des Larynx, bestehend aus einem Gerüst (2) mit zwei stabilen, zueinander im wesentlichen parallelen Bügeln (3a, 3b), einer Gaumenplatte (8) und einem Zungenspatel (15; 46a, 46b; 49; 52), wobei die Bügel (3a, 3b) durch einen Quersteg (6) zu einem U-förmigen Befestigungsgerüst (2) miteinander verbunden sind, an dem Untersuchungs- und/oder Operationswerkzeuge festlegbar sind, und wobei das Zungenspatel (15) in einem Aufnahmemittel (20;32) verschiebbar geführt ist, das an einem Ende einer Säule (21) angeordnet ist, die in einem rohr- oder hülsenförmigen Element (22) des Querstegs (6) verschiebbar (19) geführt ist, **gekennzeichnet durch** folgende Merkmale:
a) die Position des vorderen Teils (16) des Zungenspatels (15) ist gegenüber der Gaumenplatte (8) und dem U-förmigen Befestigungsgerüst (2) über einen Getriebemechanismus manvell verstellbar;
b) der Getriebemechanismus ist **durch** eine Zahnreihe und ein damit kämmendes Ritzel gebildet;
c) die Zahnreihe wird von dem Lagerungselement für das Ritzel zumindest teilweise umgriffen.

2. Vorrichtung nach Anspruch 1, **dadurch gekennzeichnet, daß**
a) die Zahnreihe an einer Zahnstange (28) angeordnet ist; und
b) das Ritzel (115) an der die Zahnstange (28) zumindest teilweise umgebenden Säule (21) gelagert ist.

3. Vorrichtung nach Anspruch 1 oder 2, **dadurch gekennzeichnet, daß**
a) die Zahnreihe (44) an dem Zungenspatel (15) angeordnet ist; und
b) das Ritzel (116) an dem den Zungenspatel (15) zumindest teilweise umgebenden Aufnahmemittel (20) gelagert ist.

4. Vorrichtung nach einem der vorhergehenden Ansprüche, **dadurch gekennzeichnet, daß** das Führungselement (22) für die den Zungenspatel (15) tragende Säule (21) im wesentlichen mittig an dem Quersteg (6) des U-förmigen Befestigungsgerüsts (2) angeordnet ist, so daß das Befestigungsgerüst (2) zusammen mit der Säule (21) je nach deren Verschiebestellung (19) die Form eines Y oder eines Dreizacks bildet.

5. Vorrichtung nach einem der vorhergehenden Ansprüche, **gekennzeichnet durch** eine Vorrichtung (25, 26) zur Arretierung der das Aufnahmemittel (20) für den Zungenspatel (15) tragenden Säule (21) gegenüber dem Führungselement (22) des Befestigungsgerüsts (2).

6. Vorrichtung nach einem der vorhergehenden Ansprüche, **dadurch gekennzeichnet, daß** das Aufnahmemittel (20) für den Zungenspatel (15) derart angeordnet ist, daß die Längsachse des Zungenspatels (15) lotrecht zu der Grundebene des U-förmigen Befestigungsgerüsts (2) verläuft.

7. Vorrichtung nach Anspruch 6, **dadurch gekennzeichnet, daß** das Aufnahmemittel (20) für den Zungenspatel (15) gegenüber dem Quersteg (6) entlang einer zu den Befestigungsbügeln (3a, 3b) senkrechten Achse (21) verschiebbar (19) ist.

8. Vorrichtung nach einem der vorhergehenden Ansprüche, **dadurch gekennzeichnet, daß** die Befestigungsbügel (3a, 3b) einen polygonalen Querschnitt aufweisen.

9. Vorrichtung nach einem der vorhergehenden Ansprüche, **dadurch gekennzeichnet, daß** die Befestigungsbügel (3a, 3b) gegenüber dem Quersteg (6) um Drehachsen verstellbar sind, die lotrecht zu der Grundfläche des U-förmigen Befestigungsgerüsts (2) verlaufen.

10. Vorrichtung nach einem der vorhergehenden Ansprüche, **dadurch gekennzeichnet, daß** das Anschlußmittel für die Bruststütze an der dem Aufnahmemittel (20) für den Zungenspatel (15) gegenüberliegenden Stirnseite der längsverschiebbaren Säule (21) angeordnet ist.

11. Vorrichtung nach einem der vorhergehenden Ansprüche, **dadurch gekennzeichnet, daß** das Anschlußmittel für eine Bruststütze mit dem Quersteg (6) des Befestigungsgerüsts (2) über einen Fortsatz verbunden ist, der lotrecht zu der Längsachse des Zungenspatels (15) verläuft.

12. Vorrichtung nach einem der vorhergehenden Ansprüche, **dadurch gekennzeichnet, daß** das Anschlußmittel für die Bruststütze als hohlzylindrische Buchse (32) ausgebildet ist.

13. Vorrichtung nach Anspruch 12, **gekennzeichnet durch** ein Knie- oder Gelenkstück (36) zur Realisierung eines Winkels zwischen einer Bruststütze und der gerüstseitigen Anschlußbuchse (32), mit einem Zapfen (35) zum Einstecken in die Anschlußbuchse (32) des Befestigungsgerüsts (2), der mit einer Buchse (37) zum Einstecken des Anschlußzapfens der Bruststütze in einem vorzugsweise stumpfen Winkel verbunden ist.

14. Vorrichtung nach einem der vorhergehenden Ansprüche, **dadurch gekennzeichnet, daß** die Gaumenplatte (8) einen Bügel (9) mit endseitig angeordneten Buchsen oder Hülsen (12) zum Aufstecken auf die freien Enden (13) des U-förmigen Befestigungsgerüsts (2) umfasst.

15. Vorrichtung nach Anspruch 14, **dadurch gekennzeichnet, daß** der Bügel (9) beidseits nach unten abgekröpft (10) ist.

16. Vorrichtung nach einem der vorhergehenden Ansprüche, **gekennzeichnet durch** einen oder mehrere Mundwinkelspatel (56a, 56b) mit einer langgestreckten, schmalen Grundform und einer abgerundeten Vorderkante (58).

17. Vorrichtung nach Anspruch 16, **dadurch gekennzeichnet, daß** der Mundwinkelspatel (56a, 56b) aus einem biegsamen Werkstoff gefertigt ist.

18. Vorrichtung nach einem der vorhergehenden Ansprüche, **gekennzeichnet durch** ein Mittel (61a; 61b) zur Aufnahme eines Mundwinkelspatels (56a; 56b) umfassend ein hülsenförmiges Element (62), welches einen Befestigungsbügel (3a; 3b) des Befestigungsgerüsts (2) umgreift, ein klammerartiges Element (66) mit einem Schlitz (63) zum Einlegen eines Mundwinkelspatels (56a; 56b), und ein zwischen Hülse (62) und Klammer (66) angeordnetes Gelenk- (65) und/oder Verstellmittel (68) zum Aufspreizen (67) des Mundwinkelspatels (56a, 56b).

19. Vorrichtung nach einem der vorhergehenden Ansprüche, **gekennzeichnet durch** ein Leuchtmittel (78) mit einer Klammer (83) zur Festlegung an einem Befestigungsbügel (3a; 3b) des Befestigungsgerüsts (2).

20. Vorrichtung nach einem der vorhergehenden Ansprüche, **gekennzeichnet durch** ein Mittel (91; 111) zur Aufnahme einer Absaugeinrichtung (104), eines Untersuchungs- oder eines Operationsinstruments (86), umfassend ein klammerartiges Element (100; 113) mit einem Schlitz (101) zum Einlegen der betreffenden Einrichtung (104) oder des betreffenden Instruments (86), sowie eine Klammer (92; 112) zur Festlegung an einem Befestigungsbügel (3a; 3b) des Befestigungsgerüsts (2).

21. Vorrichtung nach einem der vorhergehenden Ansprüche 18 bis 20, **dadurch gekennzeichnet, daß** die Befestigungsklammer (83; 92; 112) und/oder das geschlitzte (101) Klammerelement (100; 113) mit einem federnden Element (94, 95; 103) versehen ist.

22. Vorrichtung nach einem der Ansprüche 18 bis 21, **dadurch gekennzeichnet, daß** zwischen der Befestigungsklammer (83; 92) und dem Leuchtmittel (78) oder dem geschlitzten (101) Klammerelement (100) ein Kugelgelenk (98) angeordnet ist.

23. Vorrichtung nach einem der vorhergehenden Ansprüche, **dadurch gekennzeichnet, dass** das Zungenspatel (15; 46a, 46b; 49; 52) einen löffelartigen Zungenteil (16; 47a, 47b; 51) und einen daran anschließenden, geradegestreckten Halterungsteil (18; 53) aufweist, der nicht oder nur geringfügig gegenüber dem Zungenteil (16; 47a, 47b; 51) geneigt ist, wobei der gesamte Zungenspatel (15; 46a, 46b; 49; 52) aus einem steifen und festen Werkstoff gefertigt ist, und wobei die Zahnreihe (44) an dem Halterungsteil (18; 53) angeordnet sind.

24. Vorrichtung nach Anspruch 23, **dadurch gekennzeichnet, daß** das Zungenteil (47a, 47b) des Zungenspatels (15; 46a, 46b; 49; 52) mit einem seitlichen Ausschnitt (48a, 48b) versehen ist, um Untersuchungen und Operationen im Zungenbereich zu ermöglichen.

25. Vorrichtung nach Anspruch 23 oder 24, **dadurch gekennzeichnet, daß** das Zungenteil (51) des Zungenspatels (15; 46a, 46b; 49; 52) mit einer Wölbung in Querrichtung versehen ist, derart, daß die Seitenbereiche (55) des Zungenteils (51) von der Zunge nach oben divergieren.

## Claims

1. A device (1) for endoscopic examinations and/or operations in the region of the epipharynx, oropharynx, mesopharynx or hypopharynx or of the larynx, comprising a framework (2) with two stable frame-elements (3a, 3b) that are approximately parallel to one another, a palatal plate (8) and a lingual spatula (15; 46a, 46b; 49; 52), wherein the two attachment frame-elements (3a, 3b) are connected to one another by means of a crosspiece (6) so as to form an U-shaped attachment framework (2), to which examination and/or operation tools can be secured, and wherein the lingual spatula (15) is guided displaceably within a receiving means (20;32) arranged at one end of a column (21) which itself is guided displaceably (19) in a tube-shaped or sleeve-shaped element (22) of the crosspiece (6),
**characterized by** the following features:
a) the position of the front part (16) of the lingual spatula (15) is manually adjustable in relation to the palatal plate (8) and the U-shaped attachment framework (2) by means of a gearing mechanism;
b) the gearing mechanism comprises several teeth arranged in a row and meshing with a pinion;
c) the row of teeth is at least partially surrounded by a bearing means of the pinion.

2. The device as claimed in claim 1, wherein
a) the row of teeth is arranged along a toothed rack (28); and
b) the pinion (115) is supported in a rotatable manner by the column (21) which at least partially surrounds the toothed rack (28).

3. The device as claimed in claim 1 or 2, wherein
a) the row of teeth is arranged along the lingual spatula (15); and
b) the pinion (115) is supported in a rotatable manner by the receiving means (20) which at least partially surrounds the lingual spatula (15).

4. The device as claimed in one of the preceding claims, wherein the guide element (22) for the column (21) bearing the lingual spatula (15) is arranged in substantially centric manner on the crosspiece (6) of the U-shaped attachment framework (2) so that the attachment framework (2) together with the column (21) forms, depending on the displacement position (19) thereof, the shape of a Y or of a trident.

5. The device as claimed in one of the preceding claims, comprising a device (25, 26) for locking the column (21) bearing the receiving means (20) for the lingual spatula (15) in relation to the guide element (22) of the attachment framework (2).

6. The device as claimed in one of the preceding claims, wherein the receiving means (20) for the lingual spatula (15) is arranged in such a way that the longitudinal axis of the lingual spatula (15) extends approximately perpendicular to the base plane of the U-shaped attachment framework (2).

7. The device as claimed in claim 6, wherein the receiving means (20) for the lingual spatula (15) is displaceable in relation to the crosspiece (6) along an axis (21) which is approximately perpendicular to the attachment frame-elements (3a, 3b).

8. The device as claimed in one of the preceding claims, wherein the attachment frame-elements (3a, 3b) have a polygonal cross-section.

9. The device as claimed in one of the preceding claims, wherein the attachment frame-elements (3a, 3b) are displaceable in relation to the crosspiece (6) about axes of rotation that extend perpendicular to the base surface of the U-shaped attachment framework (2).

10. The device as claimed in one of the preceding claims, wherein the connecting means for the chest support is arranged on the end face of the longitudinally displaceable column (21) located opposite the receiving means (20) for the lingual spatula (15).

11. The device as claimed in one of the preceding claims, wherein the connecting means for a chest support is connected to the crosspiece (6) of the attachment framework (2) via a prolongation which extends perpendicular to the longitudinal axis of the lingual spatula (15).

12. The device as claimed in one of the preceding claims, wherein the connecting means for the chest support takes the form of a hollow cylindrical bushing (32).

13. The device as claimed in claim 12, comprising an angle piece or articulation piece (36) for realizing an angle between a chest support and the framework-side connection bushing (32) with a peg (35) for insertion into the connecting bushing (32) of the attachment framework (2), said peg being connected to a bushing (37) for insertion of the connecting peg of the chest support at a preferably obtuse angle.

14. The device as claimed in one of the preceding claims, wherein the palatal plate (8) comprises a frame-element (9) with bushings and/or sleeves (12) arranged at its end for attaching onto the free ends (13) of the U-shaped attachment framework (2).

15. The device as claimed in claim 14, wherein the frame-element (9) is cranked (10) downwards on both sides.

16. The device as claimed in one of the preceding claims, comprising a mouth corner spatula (56a, 56b), comprising one or more mouth corner spatulas (56a, 56b) with an elongated, narrow basic shape and a rounded front edge (58).

17. The device as claimed in claim 16, wherein a mouth corner spatula (56a, 56b) is produced from a flexible material.

18. The device as claimed in one of the preceding claims, comprising a means (61a; 61b) for receiving a mouth corner spatula (56a; 56b) with a sleeve-shaped element (62) which encompasses an attachment frame-element (3a; 3b) of the attachment framework (2), a clip-like element (66) with a slot (63) for insertion of a mouth corner spatula (56a; 56b), and an articulation means (65) and/or adjusting means (68) arranged between sleeve (62) and clip (66) for spreading the mouth corner spatula (56a, 56b) apart.

19. The device as claimed in one of the preceding claims, comprising a lighting means (78) with a clip (83) for securing to an attachment frame-element (3a; 3b) of the attachment framework (2).

20. The device as claimed in one of the preceding claims, comprising a means (91; 111) for receiving a suction device (104), an examining instrument and /or a surgical instrument (86) with a clip-type element (100; 113) with a slot (101) for insertion of the device (104) in question and/or of the instrument (86) in question and also a clip (92; 112) for securing to an attachment frame-element (3a; 3b) of the attachment framework (2).

21. The device as claimed in one of claims 18 to 20, wherein the attachment clip (83; 92; 112) and/or the slotted (101) clip element (100;113) is provided with a springing element (94, 95; 103).

22. The device as claimed in one of claims 18 to 21, wherein a ball-and-socket joint (98), is arranged between the attachment clip (83; 92) and the lighting means (78) or the slotted (101) clip element (100).

23. The device as claimed in one of the preceding claims, comprising a spoon-type tongue part (16; 47a, 47b; 51) and a straight holding part (18; 53) adjacent thereto which is not inclined or only slightly inclined in relation to the tongue part (16; 47a, 47b; 51), the entire lingual spatula (16; 46a, 46b; 49; 52) being produced from a rigid and solid material, and whereby the row of teeth (44) is arranged at the holding part (18; 53).

24. The device as claimed in claim 23, wherein the tongue part (47a, 47b) of the lingual spatula (46a, 46b) is provided with a lateral cut-out (48a, 48b) in order to enable examinations and operations in the lingual region.

25. The device as claimed in claim 23 or 24, wherein the tongue part (51) of the lingual spatula (15;46a,46b;4952) is provided with a curvature in the transverse direction in such a way that the lateral regions (55) of the tongue part (51) diverge upwards from the tongue.

## Revendications

1. Dispositif (1) pour des examens endoscopiques, et/ou des opérations dans la zone de l'épipharynx, de l'oropharynx, du mésopharynx ou de l'hypopharynx ou du larynx, comportant une ossature (2) munie de deux éléments de châssis stables (3a, 3b) qui sont approximativement parallèles l'un à l'autre, une plaque palatale (8) et une spatule linguale (15; 46a, 46b; 49; 52), les deux éléments de châssis de fixation (3a, 3b) étant assemblés l'un à l'autre par l'intermédiaire d'une traverse (6) de manière à former une ossature de fixation en forme de U (2) sur laquelle on peut fixer des outils d'examen et/ou d'opération, et la spatule linguale (15) étant guidée de manière mobile à l'intérieur de moyens de réception (20; 32) disposés à une première extrémité d'une colonne (21) qui elle-même est guidée de manière mobile (19) dans un élément en forme de tube ou en forme de manchon (22) de la traverse (6), **caractérisé par** les caractéristiques suivantes:
a) la position de la partie avant (16) de la spatule linguale (15) peut être ajustée manuellement par rapport à la plaque palatale (8) et à l'ossature de fixation en forme de U (2) par l'intermédiaire d'un mécanisme à engrenages,
b) le mécanisme à engrenages comporte plusieurs dents agencées en un rang et engrenant avec un pignon,
c) le rang de dents est au moins partiellement entouré par des moyens formant palier du pignon.

2. Dispositif selon la revendication 1, dans lequel
a) le rang de dents est disposé le long d'une crémaillère dentée (28), et
b) le pignon (115) est supporté d'une manière rotative par la colonne (21) qui entoure au moins partiellement la crémaillère dentée (28).

3. Dispositif selon la revendication 1 ou 2, dans lequel
a) le rang de dents est disposé le long de la spatule linguale (15), et
b) le pignon (115) est supporté de manière rotative par les moyens de réception (20) qui entourent au moins partiellement la spatule linguale (15).

4. Dispositif selon l'une quelconque des revendications précédentes, dans lequel l'élément de guidage (22) de la colonne (21) supportant la spatule linguale (15) est disposé d'une manière sensiblement centrale sur la traverse (6) de l'ossature de fixation en forme de U (2), de sorte que l'ossature de fixation (2) forme en association avec la colonne (21), selon la position de déplacement (19) de celle-ci, la forme d'un Y ou d'un trident.

5. Dispositif selon l'une quelconque des revendications précédentes, comportant un dispositif (25, 26) pour verrouiller la colonne (21) supportant les moyens de réception (20) de la spatule linguale (15) par rapport à l'élément de guidage (22) de l'ossature de fixation (2).

6. Dispositif selon l'une quelconque des revendications précédentes, dans lequel les moyens de réception (20) de la spatule linguale (15) sont disposés d'une manière telle que l'axe longitudinal de la spatule linguale (15) s'étend approximativement perpendiculairement au plan de base de l'ossature de fixation en forme de U (2).

7. Dispositif selon la revendication 6, dans lequel les moyens de réception (20) de la spatule linguale (15) peuvent être déplacés par rapport à la traverse (6) le long d'un axe (21) qui est approximativement perpendiculaire aux éléments de châssis de fixation (3a, 3b).

8. Dispositif selon l'une quelconque des revendications précédentes, dans lequel les éléments de châssis de fixation (3a, 3b) ont une coupe transversale polygonale.

9. Dispositif selon l'une quelconque des revendications précédentes, dans lequel les éléments de châssis de fixation (3a, 3b) peuvent être déplacés par rapport à la traverse (6) autour d'axes de rotation qui s'étendent perpendiculairement à la surface de base de l'ossature de fixation en forme de U (2).

10. Dispositif selon l'une quelconque des revendications précédentes, dans lequel les moyens de connexion destinés à un support de poitrine sont disposés sur une face d'extrémité de la colonne mobile longitudinalement (21) située à l'opposé des moyens de réception (20) de la spatule linguale (15).

11. Dispositif selon l'une quelconque des revendications précédentes, dans lequel les moyens de connexion destinés à un support de poitrine sont connectés à la traverse (6) de l'ossature de fixation (2) via un prolongement qui s'étend perpendiculairement à l'axe longitudinal de la spatule linguale (15).

12. Dispositif selon l'une quelconque des revendications précédentes, dans lequel les moyens de connexion destinés à un support de poitrine prennent la forme d'une bague cylindrique creuse (32).

13. Dispositif selon la revendication 12, comportant une pièce d'angle ou une pièce d'articulation (36) destinée à réaliser un angle entre un support de poitrine et la bague de connexion côté ossature (32) à l'aide d'une cheville (35) d'insertion dans la bague de connexion (32) de l'ossature de fixation (2), ladite cheville étant connectée à une bague (37) d'insertion de la cheville de connexion du support de poitrine au niveau d'un angle de préférence obtus.

14. Dispositif selon l'une quelconque des revendications précédentes, dans lequel la plaque palatale (8) comporte un élément de châssis (9) muni de manchons et/ou de bagues (12) agencés au niveau de son extrémité pour une fixation sur les extrémités libres (13) de l'ossature de fixation en forme de U (2).

15. Dispositif selon la revendication 14, dans lequel les éléments de châssis (9) sont coudés (10) vers le bas des deux côtés.

16. Dispositif selon l'une quelconque des revendications précédentes, comportant une spatule de coin de bouche (56a, 56b), comportant une ou plusieurs spatules de coin de bouche (56a, 56b) ayant une forme fondamentale étroite allongée et un bord avant arrondi (58).

17. Dispositif selon la revendication 16, dans lequel la spatule de coin de bouche (56a, 56b) est fabriquée à partir d'un matériau souple.

18. Dispositif selon l'une quelconque des revendications précédentes, comportant des moyens (61a; 61b) destinés à recevoir une spatule de coin de bouche (56a; 56b) à l'aide d'un élément en forme de manchon (62) qui entoure un élément de châssis de fixation (3a; 3b) de l'ossature de fixation (2), un élément analogue à une attache (66) ayant une fente (63) pour insertion d'une spatule de coin de bouche (56a; 56b), et des moyens d'articulation (65) et/ou des moyens d'ajustement (68) disposés entre le manchon (62) et l'attache (66) pour écarter la spatule de coin de bouche (56a, 56b).

19. Dispositif selon l'une quelconque des revendications précédentes, comportant des moyens d'éclairage (78) munis d'une attache (83) pour se fixer à un élément de châssis de fixation (3a; 3b) de l'ossature de fixation (2).

20. Dispositif selon l'une quelconque des revendications précédentes, comportant des moyens (91; 111) destinés à recevoir un dispositif d'aspiration (104), un instrument d'examen et/ou un instrument chirurgical (86) munis d'un élément de type attache (100; 113) ayant une fente (101) pour insertion du dispositif (104) en question et/ou de l'instrument (86) en question, et également d'une attache (92; 112) pour une fixation sur un élément de châssis de fixation (3a; 3b) de l'ossature de fixation (2).

21. Dispositif selon l'une quelconque des revendications 18 à 20, dans lequel l'attache de fixation (83; 92; 112) et/ou l'élément d'attache (100; 113) muni d'une fente (101) sont munis d'un élément formant ressort (94, 95; 103).

22. Dispositif selon l'une quelconque des revendications 18 à 21, dans lequel un joint à rotule sphérique (98) est disposé entre l'attache de fixation (83; 92) et les moyens d'éclairage (78) ou l'élément d'attache (100) muni d'une fente (101).

23. Dispositif selon l'une quelconque des revendications précédentes, comportant une partie formant languette de type cuiller (16; 47a, 47b; 51) et une partie de support rectiligne (18; 53) adjacente à celle-ci qui n'est pas inclinée ou seulement légèrement inclinée par rapport à la partie de languette (16; 47a, 47b; 51), toute la spatule linguale (16; 46a, 46b; 49; 52) étant produite à partir d'un matériau rigide et solide, et de sorte que le rang de dents (44) est disposé au niveau de la partie de support (18; 53).

24. Dispositif selon la revendication 23, dans lequel la partie formant languette (47a, 47b) de la spatule linguale (46a, 46b) est munie d'une découpe latérale (48a, 48b) pour permettre des examens et des opérations dans la région linguale.

25. Dispositif selon la revendication 23 ou 24, dans lequel la partie formant languette (51) de la spatule linguale (15; 46a, 46b; 49; 52) est munie d'une courbure dans la direction transversale d'une manière telle que les régions latérales (55) de la partie formant languette (51) divergent vers le haut à partir de la languette.
